# EUROPÄISCHE PATENTSCHRIFT

(11) **EP 4 248 184 B1**
(45) Veröffentlichungstag und Bekanntmachung des Hinweises auf die Patenterteilung: **31.12.2025**
(21) Anmeldenummer: 21820468.3
(22) Anmeldetag: 18.11.2021
(51) Int. Cl.: G01K 13/20, G01K 1/14, G01K 1/024, A61B 5/01, A61B 5/00, A61B 5/0205, A61B 5/22

(54) **SENSORVORRICHTUNG ZUR BESTIMMUNG VON ENTZÜNDUNGSRELEVANTEN VITALPARAMETERN**
SENSOR DEVICE FOR THE DETERMINATION OF INFLAMMATORY VITAL PARAMETERS
DISPOSITIF CAPTEUR PERMETTANT DE DÉTERMINER DES PARAMÈTRES VITAUX LIÉS À UNE INFLAMMATION

(30) Priorität: 20.11.2020 EP 20209022
(43) Veröffentlichungstag der Anmeldung: 27.09.2023
(73) Patentinhaber: Geffe, Markus, 47198 Duisburg (DE)
(72) Erfinder: Geffe, Markus, 47198 Duisburg (DE)
(74) Vertreter: Seyer & Nobbe Patentanwälte PartmbB
(86) Internationale Anmeldenummer: PCT/EP2021/082226
(87) Internationale Veröffentlichungsnummer: WO 2022/106583

(56) Entgegenhaltungen:
- EP-B1- 3 075 312
- DE-U1- 29 715 113
- US-B1- 6 846 106
- US-B2- 8 444 622
- TURK VICTORIA: "This sleep-tracking Oura ring can detect when you've drunk too much", WIRED UK, 18 June 2019 (2019-06-18), Vogue House 1-2 Hanover Sq London W1S 1JU (UK), pages 1 - 9, XP055972985, Retrieved from the Internet <URL:https://www.wired.co.uk/article/oura-ring-uk-sleep-tracking> [retrieved on 20221019]

## Beschreibung

Die vorliegende Erfindung betrifft eine Sensorvorrichtung zur Bestimmung von entzündungsrelevanten Vitalparametern.

Eine Entzündung ist eine körpereigene Reaktion auf schädliche Reize, die sich klassischerweise durch die Entzündungszeichen wie Rötung, Schwellung, Schmerz und funktionelle Einschränkungen äußert. Botenstoffe des Immunsystems bewirken dabei eine Erweiterung der Blutgefäße, sodass das Entzündungsgebiet stärker durchblutet wird und die Gefäße durchlässiger für den Austritt von Blutplasma und Immunzellen ins Gewebe werden. Jeder das physiologische Maß übersteigende Reiz kann eine Entzündung auslösen. Dies gilt insbesondere für physikalische Reize wie mechanische Reize oder Fremdkörper wie zum Beispiel Stoffwechselprodukte wie Harnsäurekristalle, sowie für Allergene und Autoallergene zum Beispiel bei rheumatischen oder Autoimmunerkrankungen.

Harnsäurekristalle werden beispielsweise infolge einer hohen Harnsäurekonzentration im Blut beispielsweise in verschiedenen peripheren Gelenken und Geweben abgeschieden. Durch Ablagerungen der Harnsäurekristalle kommt es zu einer gelenksnahen Knochenresorption und Knorpelveränderungen, die sich im Krankheitsbild einer Gichtarthritis niederschlagen. Dabei ist das Gelenk in der Regel stark gerötet, extrem schmerzhaft, stark geschwollen und überwärmt.

Ähnliche Symptome werden bei rheumatischen Erkrankungen beobachtet. Dabei greift der Körper eigene Strukturen wie die Gelenkinnenhaut, etwa bei der rheumatoiden Arthritis, an und infolge der zumeist chronischen Entzündungen leiden die Betroffenen gelenkbezogener Formen (Gelenkrheumatismus, bzw. chronischer Gelenkrheumatismus) unter Schmerzen, Schwellungen oder Ergüssen der Gelenke sowie als Spätfolgen unter Gelenkszerstörung, Fehlstellungen und Funktionsverlust.

Der Verlauf einer rheumatischen Erkrankung und das Ansprechen auf eine Therapie können selbst bei gleicher Diagnose von Patient zu Patient äußerst unterschiedlich ausfallen. Entscheidend für den Verlauf einer rheumatischen Erkrankung ist dabei oftmals die rechtzeitige Diagnose und Therapie, um den zuvor beschriebenen Spätfolgen vorzubeugen. Dazu kommt es insbesondere darauf an, an den betroffenen Gelenken frühzeitig erste Symptome einer rheumatischen Erkrankung oder deren Wiederaufflammen festzustellen und rechtzeitig eine Therapie einleiten zu können.

Zu den ersten Symptomen einer Gichterkrankung oder rheumatischen Erkrankung gehört die Temperaturerhöhung des betroffenen Gelenkes sowie ein Anschwellen des Gelenkes über die normale Größe hinaus. Wenn diese Symptome frühzeitig erkannt werden, ist entsprechend die Einleitung einer Therapie ebenso frühzeitig möglich. Somit können dann medikamentöse Therapien eingeleitet werden, die insbesondere mit schmerzlindernden und entzündungshemmenden Medikamenten durchgeführt werden. Aufgrund der deutlichen Risiken und möglichen Nebenwirkungen muss allerdings besonders bei einer längerfristigen Verordnung jeweils eine individuelle Abschätzung und Indikation möglicher Risiken erfolgen.

In der wissenschaftlichen Forschung solcher Erkrankungen der Gelenke wie Gicht und rheumatoider Arthritis sind Überlegungen angestellt worden, inwieweit der Krankheitsverlauf durch Bestimmung der Temperaturprofile der Erkrankten an den betroffenen Gelenken aufgezeichnet werden kann. Im Stand der Technik ist jedoch keine Vorrichtung bekannt, mit der auf einfache und für den Patienten bequeme Art und Weise der Temperaturverlauf, insbesondere der betroffenen Gelenke, zuverlässig aufgezeichnet werden kann.

Aus der DE 29715113U1, ähnlich der US 6 846 106 B1, ist ein Fingertemperatur-Anzeigering bekannt, der zuverlässig und unabhängig von der psychischen Stimmung des Patienten die Körpertemperatur bestimmen soll. Dieser Ring wird wie ein normaler Ring zwischen den Gelenken beispielsweise des Ringfingers getragen. Die Messung der Fingertemperatur wird bei dieser Vorrichtung direkt neben dem Ringkörper und so möglichst unauffällig durchgeführt, um die Schwankungen der Körpertemperatur infolge psychischer Belastung auszuschließen. Eine Messung der Gelenktemperatur oder anderer Parameter ist gemäß dieser Druckschrift nicht vorgesehen.

Im Stand der Technik sind eine Reihe von Vorrichtungen bekannt, die zur Bestimmung physiologischer Parameter am Finger oder anderen Gliedmaßen des Menschen bestimmt sind.

So beschreibt die DE 20 2006 009 103 ein handschuhartiges biologisches Messgerät, das aus einem handschuhartigen Hauptkörper, der von dem Benutzer angezogen werden kann und elektrische Anschlüsse aufweist, wobei an der Stelle eines Fingers eine Öffnung vorgesehen ist, an der ein biologisches Messgerät angeordnet werden kann. Als ein solches biologisches Messgerät wird ein Lichtsender und ein Lichtempfänger vorgeschlagen, die zur Messung des Sauerstoffgehaltes im Blut dienen.

In der EP 2437039A2 wird ein ergonomisches Thermometer beschrieben, dass in der Hand gehalten wird und zur Bestimmung der Temperatur der Hautoberfläche vorgesehen ist. Nachteilig an dieser Vorrichtung ist die Notwendigkeit, dass das Thermometer konstant unter Druck zwischen den Fingern getragen werden muss.

Die EP 3075312B1 offenbart eine ringförmige Vorrichtung zur Messung des Hautwiderstandes des Patienten, wobei zwischen einer inneren Elektrode und einer äußeren Elektrode der Hautwiderstand des Anwenders gemessen wird.

Aus der WO 2018/073827A1 ist eine Vorrichtung und ein System zur Aufzeichnung von physiologischen Signalen von einem Finger bekannt, wobei die Vorrichtung einen Körper mit einer Fingerhut-artigen Spitze ist, die mit eines ein die mithilfe eines Bandes am Finger festgelegt wird und physiologische Bedingungen wie Sauerstoffsättigung des Blutes bestimmen soll.

Allen vorgenannten Vorrichtungen ist gemein, dass diese nicht zur Messung der Temperatur an einem Gelenk eines Fingers oder eines Zehs geeignet sind.

Es besteht daher ein Bedarf an einer Vorrichtung, mit der sich der Temperaturverlauf an einem Gelenk eines Fingers oder Zehs zuverlässig und sicher über einen längeren Zeitraum bestimmen lässt und so die Möglichkeit eröffnet, die Therapie, insbesondere die Verminderung des Schmerzempfinden des Patienten so einzuleiten, dass der Patient mit niedrigeren Dosen der Therapeutika behandelt werden kann und somit eine Verringerung der Langzeitschäden der medikamentösen Therapie erzielt wird.

Seitens des Erfinders der vorliegenden Erfindung wurde daher eine Vorrichtung entwickelt, mit der sich auf insbesondere für den Patienten einfache und bequeme Weise die Temperatur eines Gelenkes oder mehrerer Gelenke zuverlässig und kontinuierlich messen lässt, ohne dass der Patient dabei besonderen Einschränkungen hinsichtlich seiner Beweglichkeit unterworfen ist.

In einer Weiterentwicklung der erfindungsgemäßen Vorrichtung lässt sich der Temperaturverlauf mehrerer Gelenke an einem Finger oder mehreren Fingern, und/oder Zehen, protokollieren. Ebenso ist es gemäß einer Weiterbildung möglich, die Zunahme der Fingerdicke durch Anschwellen zu bestimmen.

Die vorliegende Erfindung ist daher gerichtet auf eine Vorrichtung zur Bestimmung der Temperatur eines Gelenkes einer menschlichen Hand oder eines menschlichen Zehs in Form eines ringförmigen Sensorsystems.

So betrifft die vorliegende Erfindung eine Sensorvorrichtung zur Bestimmung von entzündungsrelevanten Vitalparametern, mit einem Grundkörper mit einer im Wesentlichen rechteckigen Grundfläche, einem am Grundkörper festgelegten ringförmigen Halter zum Aufstecken auf einen Finger eines Patienten und mindestens einem am Grundkörper festgelegten Sensorarm, wobei eine einem Temperatursensor funktionell zugeordnete Sensorfläche am Sensorarm endständig so angeordnet ist und der Sensorarm eine solche Länge aufweist, dass die Sensorfläche in der Gebrauchsposition auf dem Fingergrundgelenk des Patienten zur Anlage kommt und wobei im Grundkörper Mittel zur Speicherung und zur optionalen Übertragung der von dem Temperatursensor gemessenen Daten auf eine Datenauswerteeinheit vorgesehen sind.

Die erfindungsgemäße Sensorvorrichtung besteht in der einfachsten Form aus einem Grundkörper mit daran angesetztem ringförmigen Halter. Der Grundkörper, der zur Aufnahme der elektronischen Bauteile wie Datenspeicher, Sende- und Empfangseinrichtung, Lithium-Ionen-Batterie, etc, ausgelegt ist, kann im Wesentlichen die Form eines Quaders mit einer jeweils teilkreisförmig gebogenen Grundfläche und Deckfläche haben, in den die elektronischen Bauelemente entsprechend integriert sind. Der mindestens eine Sensorarm ist vorzugsweise an der zum Fingergrundgelenk gerichteten Seite des Grundkörpers angeordnet und in seiner Länge so bemessen, dass das endseitig am Sensorarm angeordnete Sensorelement in Form eines Temperatursensors über die Sensorfläche auf dem Fingergrundgelenk zur Anlage kommt. Dabei kann der Sensorarm, je nach Ausführung der erfindungsgemäßen Vorrichtung, ein starrer Arm sein oder nach Art eines Teleskopstabes zwischen einer Anfangs- und Endposition verschiebbar sein, sodass die Länge entsprechend den physiognomischen Gegebenheiten des Fingers eingestellt werden kann. Entsprechendes gilt für den in einer weiteren Ausführungsform vorgesehenen zweiten Sensorarm, der in Richtung des Fingermittelgelenkes angeordnet ist. An den Längsseiten des Grundkörpers sind vorzugsweise zwei Ringelemente wie beispielsweise Halbringe angeordnet, die den Finger umschließen. Vorzugsweise sind die Ringsegmente/Halbringe in ihrer Breite auf die Breite der Längsseite des Grundkörpers abgestimmt und die beiden umlaufenden Kanten des ringförmigen Halters sind in Richtung des Fingermittelgelenkes angeschrägt. Auf diese Weise wird die Breite der Längsseite des Grundkörpers etwa beibehalten und ein kippneigungsfreier Sitz der Sensorvorrichtung am Finger sichergestellt.

In diesen Ringsegmenten können vorzugsweise weitere Temperatursensoren an der auf der Haut des Fingers aufliegenden Innenseite angeordnet sein, um die Messpunktanzahl am Finger zu erhöhen und somit mit einer vergrößerten Datenbasis genauere Diagnosen bzw. datenbasierte Handlungsempfehlungen ableiten zu können. Die Messpunkte in den Ringsegmenten sind vorzugsweise entlang der radialen Spiegelebene zwischen den jeweils zum Gelenk ausgerichteten Sensorarmen angebracht. Alternativ können diese auch symmetrisch zwischen den Sensorarmen oder in weiteren gegebenenfalls anwenderspezifischen Bereichen in den Ringsegmenten angeordnet sein. Die Anzahl von Messpunkten in den Ringsegmenten kann anwendungsspezifisch ausgelegt sein.

Das Material der erfindungsgemäßen Sensorvorrichtung ist nicht entscheidend, solange die Funktionsweise der Vorrichtung nicht beeinträchtigt ist. Besonders vorteilhaft können die einzelnen Bauteile aus Kunststoff durch 3D-Druck oder durch Spritzgussverfahren hergestellt werden. Die erfindungsgemäße Sensorvorrichtung kann auch in einen Handschuh integriert sein, was die Verwendbarkeit für manche Patienten erleichtern kann. Die erfindungsgemäße Sensorvorrichtung kann in ein wasser- und staubdichtes Gehäuses integriert sein, in dem die elektronischen Bauteile zur Erfassung, Speicherung, Auswertung und Datenkommunikation vor Wasser und Staub geschützt sind. Alternativ kann eine wasser- und staubdicht eingekapselte Elektronik verwendet werden, die dann auch die Verwendung eines nicht wasserdichten Gehäuses erlaubt.

Erfindungsgemäß ist der mindestens eine am Grundkörper angeordnete Sensorarm über ein Zwischenelement am Grundkörper lösbar festgelegt. Das Zwischenelement kann in Form eines flexiblen Elementes, das einer Auslenkung aus der Ausgangsposition mit einer Rückstellkraft entgegenwirkt, etwa nach Art einer flexiblen Unterlegscheibe, ausgebildet sein. Das Zwischenelement kann am Grundkörper und/oder der Grundkörper am Zwischenelement ebenfalls lösbar befestigt sein. So wird ermöglicht, dass Sensorarme in verschiedener Länge und Zwischenelemente unterschiedlicher Dicke an einem Grundkörper festlegbar sind und auf die physiognomischen Verhältnisse beim Patienten wie Finger- oder Zehengliedlänge abgestimmt werden können.

Mithilfe des Zwischenelementes kann erreicht werden, dass das Sensorelement über die Sensorfläche auch bei Bewegung des Fingers auf dem jeweiligen Gelenk anliegt, wobei die Rückstellkraft des Zwischenelementes, z.B. als Federelement, dafür Sorge trägt. Des Weiteren kann durch das Zwischenelement in Form eines Armfederelementes erreicht werden, dass die Anpresskraft der Sensorfläche auf der Anliegefläche des jeweiligen Finger- oder Zehgelenkes eingestellt werden kann. Durch Nutzung sehr flexibler Werkstoffe im Armfederelement kann die o.g. Anpresskraft auch erhöht werden, bzw. durch Nutzung sehr steifer Werkstoffe im Armfederelement kann die Anpresskraft verringert werden. Somit kann eine individuelle patientenspezifische Anpassung der Anpresskraft der Fläche des Sensorarms in Bewegungsrichtung orthogonal zur Anliegefläche des Sensors erfolgen.

In einer weiteren Ausführung kann am Grundkörper ein weiterer Sensorarm optional über ein Zwischenelement lösbar so festgelegt sein, dass eine einem Temperatursensor funktionell zugeordnete Sensorfläche am Sensorarm endständig so angeordnet ist und der Sensorarm eine solche Länge aufweist, dass die Sensorfläche in der Gebrauchsposition auf dem Fingermittelgelenk des Patienten zur Anlage kommt. Das Zwischenelement kann dabei wie das zuvor beschriebene Zwischenelement als Armfederelement ausgeführt sein. Bei der erfindungsgemäßen Sensorvorrichtung schließen der Sensorarm am Fingergrundgelenk und/oder der Sensorarm am Fingermittelgelenk zu der durch die Grundfläche des Grundkörpers aufgespannten Ebene einen Winkel von 0° bis 45°, insbesondere 20° bis 30° - im Folgenden als Anstellwinkel bezeichnet - in Richtung des ringförmigen Halters 2 ein.

In einer weiteren Ausführung der erfindungsgemäßen Sensorvorrichtung kann ein im Grundkörper vorgesehener Stellmotor dazu genutzt werden, den Anstellwinkel zu verändern. Weist die erfindungsgemäße Sensorvorrichtung zwei gegenüberliegende Sensorarme auf, können zwei Stellmotoren mit jeweils einem Stellmotor pro Sensorarm verbaut sein. Dieser Stellmotor bzw. diese Stellmotoren kann/können sowohl einseitig oder beidseitig den zuvor genannten Anstellwinkel vor, während oder nach dem Temperaturmessungszyklus der erfindungsgemäßen Sensorvorrichtung verändern. Diese Winkelveränderung kann ein komfortableres Auf- und Abziehen der erfindungsgemäßen Sensorvorrichtung ermöglichen, ferner auch eine Veränderung der Anpresskraft der Sensorfläche auf die Anliegefläche des jeweiligen Finger- oder Zehengelenkes erlauben.

Zu diesem Zweck kann der eingangs genannte Anstellwinkel auch negative Werte aufweisen, sodass ein oder beide Sensorarme von der Mittelachse des ringförmigen Halters wegzeigen.

Insbesondere zur Nutzung der erfindungsgemäßen Sensorvorrichtung an einem Zeh kann der Anstellwinkel dauerhaft negative Werte aufweisen, sodass die die Vorrichtung bequemer zu tragen ist z.B. in einem mehrstündigen Messzyklus z.B. über Nacht.

Ferne kann die Verstellung des Anstellwinkels dazu genutzt werden, die Anpresskraft der Sensorfläche auf die Anliegefläche des jeweiligen Finger- oder Zehengelenkes so weit zu erhöhen, dass der Patient bis an eine für ihn nicht mehr ertragbare Schmerzgrenze gebracht wird. Dadurch kann die Druckschmerzempfindlichkeit der Anliegefläche bestimmt werden. Eine hohe Druckschmerzempfindlichkeit korreliert i.d.R mit einer hohen Wahrscheinlichkeit für das Vorliegen einer rheumatoiden Arthritis. Diese Verstellung des Anstellwinkels mit Erhöhung der Anpresskraft kann an einen oder auch an beiden Sensorarmen zeitgleich oder zeitversetzt erfolgen.

Durch die winkelige Anordnung des oder der Sensorarme wird die Anlage der Sensorfläche an dem jeweiligen Gelenk weiter unterstützt und die Rückstellkraft der Armfederelemente ausgenutzt.

Der ringförmige Halter kann in einer weiteren Ausführungsform zwei Ringsegmente umfassen, von denen zumindest eines über ein Federelement am Grundkörper festgelegt ist.

Bei dieser Ausführungsform ist das Aufstecken des ringförmigen Halters auf den Finger erleichtert, da die beiden Ringsegmente, beispielsweise eines über ein Scharnier gelagert, gegeneinander beweglich sind.

Die Ringsegmente können an den Federelementen oder mit diesen zusammen am Grundkörper lösbar festgelegt sein Eine solche Ausführung erlaubt es, an einer Ausführung eines Grundkörpers Ringsegmentpaare mit unterschiedlichen Durchmessern festzulegen, und so die erfindungsgemäße Sensorvorrichtung in verschieden Größenvarianten dem Patienten angepasst bereitzustellen. So können mit wenigen, gegebenenfalls nur einer Grundkörper-Größenvariante alle Ringe aufgebaut werden.

Dabei kann der ringförmige Halter zwei Ringsegmente umfassen, die jeweils über ein Federelement am Grundkörper festgelegt sind und an dem, dem Grundkörper gegenüberliegenden Ende einen Spalt ausbilden, wobei die Ringsegmente jeweils an ihrem dem Spalt zugewandten Ende eine Elektrode aufweisen, an die eine elektrische Spannung unter Ausbildung eines elektrischen Potentials angelegt werden kann. Dieser Ausführungsform kann dazu verwendet werden, zusätzlich zu der Messung der Temperatur des Fingergrundgelenkes und/oder Fingermittelgelenkes die Fingerdicke bei einem Rheumaschub und/oder bei einer Schub-Prognose-Messung im Alltag zu messen. Bei zunehmender Entzündung schwillt der Finger an und die dem Grundkörper gegenüberliegenden Enden der Ringsegmente werden auseinandergedrückt. Infolge der Vergrößerung des Abstandes der Elektroden verringert sich die Kapazität des Kondensators und korreliert mit der Zunahme der Fingerdicke. Auf diese Weise kann der Therapeut sich ein umfassenderes Bild vom Entzündungszustand des Patienten machen.

Ferner kann in einer weiteren Ausführung die Fingerdicke zusätzlich zu der Messung der Temperatur des Fingergrundgelenkes und/oder Fingermittelgelenkes dadurch bestimmt werden, dass eine mit einem Ringsegment starr verbundene Fläche innerhalb des Grundkörpers mit einer starren Fläche des Grundkörpers einen Winkel ausbildet, welche sich im gleichen Betrag (jedoch entgegengesetztem Vorzeichen) wie der Winkel des Ringsegmentes ändert, gemäß einem Nebenwinkel der Geometrielehre. Dieser Winkel sei als Hilfswinkel definiert. Da der Hilfswinkel mit der Zunahme der Fingerdicke negativ korreliert (=abnimmt), kann sich auch hierdurch der Therapeut ein umfassendes Bild vom Entzündungszustand des Patienten machen.

Die erfindungsgemäße Sensorvorrichtung kann vorzugsweise auf der dem Finger oder Zeh zugewandten Seite in dem ringförmigen Halter aus den Ringsegmenten und dem Grundkörper zumindest einen weiteren Sensor zur Bestimmung eines physiologischen Parameters, ausgewählt aus Leitfähigkeit der Haut, Sauerstoffsättigung des Blutes, Puls und Herzrhythmus, Konzentration der Erythrozyten, der Leukozyten der Lymphozyten und von Hämoglobin (Hb), aufweisen.

Die erfindungsgemäße Sensorvorrichtung kann in dem ringförmigen Halter oder dem Grundkörper eine weitere Vorrichtung aufweisen, über die Messstreifen zur Analyse von aufgetragenen Bluttropfen zumindest teilweise eingeführt werden können. Dadurch können krankheitsrelevante Entzündungseiweiße, Antikörper, Harnsäure, Erreger oder weitere Blutwerte bestimmt werden, sodass der Gesundheitszustand des Patienten noch weiter erfasst werden kann und auch. das Vorhandensein einer rheumatoiden Arthritis weiter bestimmt werden kann. Typische Entzündungswerte könnten unter anderem auch so bestimmt werden: C-reaktives Protein (CRP), typische Antikörper einschließlich Antikörper gegen cyclische citrullinierte Peptide (Anti-CCP) und Rheumafaktor (RF) sowie typische organische Werte wie Alanin-Aminotransferase (ALT), Aspartat-Aminotransferase (AST) und Kreatinin (Krea).

Die erfindungsgemäße Sensorvorrichtung kann auf der dem Finger oder Zeh bei Gebrauch abgewandten Seite, auf der Außenfläche des Grundkörpers oder des ringförmigen Halters einen oder mehrere weitere Temperatursensoren aufweisen. Diese können zur Messung der Temperatur des umgebenden Mediums, welches z.B. Luft aber auch Wasser sein kann, genutzt werden. So kann die erfindungsgemäße Sensorvorrichtung über Sensoren an den Außenflächen die Temperatur an die Auswerteeinheit rückmelden, sodass eine geeignete Starttemperatur des umgebenen Mediums für einen Messzyklus festgestellt und der Start eines Messzyklus freigegeben werden kann. Dies ist bei einem sogenannten kalten Provokationstest sehr sinnvoll, da hier Finger oder Zehen auf eine definierte Temperatur heruntergekühlt werden, sodass diese mit ihrer Eigenerwärmung eine Temperaturaufwärmkurve erfassen lassen, über welche sich Prognose und Diagnose von einem rheumatoiden Krankheitsverlauf bestimmen lassen.

Neben der Bestimmung der Temperatur des oder der Fingergelenke und der Bestimmung der Fingerdicke können in dem ringförmigen Halter und oder dem Grundkörper weitere Sensoren zur Bestimmung weiterer physiologischer Parameter angeordnet sein. Auf diese Weise kann sich der Therapeut ein noch weitergehendes Bild vom Zustand des Patienten machen.

Ergänzend zu direkten physiologischen Parametern kann die erfindungsgemäße Sensorvorrichtung über ein integriertes Gyroskop und/oder einen integrierten Beschleunigungssensor verfügen. Mittels eines oder beider Sensoren können Informationen über die Steifigkeit bzw. Beweglichkeit des Finger oder Zehs gewonnen werden.

Darüber hinaus betrifft die Erfindung auch ein System zur Bestimmung von entzündungsrelevanten Vitalparametern, das zumindest zwei Sensorvorrichtungen, die miteinander zur Übertragung der von der jeweiligen Sensorvorrichtung gewonnenen und gespeicherten Daten in einer Kommunikationsverbindung gekoppelt sind, umfasst. Mithilfe dieses gekoppelten Systems ist es möglich, an mehreren Fingern die physiologischen Parameter wie Temperatur, Fingerdicke, etc. zu bestimmen und die Messwerte gegeneinander abzugleichen.

Neben zwei gekoppelten Sensorsystemen an zwei Fingern betrifft die Erfindung auch ein System von zwei gekoppelten Sensorvorrichtungen am gleichen Finger, die an den jeweiligen Stirnflächen der jeweils zugewandten Sensorarmes verbunden und gekoppelt sein können. Die beide Sensorvorrichtungen können dann über dem Mittelgliedgelenk verbunden sein, sodass der ringförmige Halter des Primärsystems das Grundglied (erstes Fingerglied) und der ringförmige Halter des Sekundärsystem das Mittelglied (zweites Glied) umschließt. Dadurch kann der zum Fingerende gerichtete Sensorarm des Sekundärsystems auf dem Fingerendgelenk analog zur zuvor beschriebenen Art unter Einstellung der Anpresskraft, etc. zur Auflage kommen.

In den Ringsegmenten der zwei gekoppelten Sensorvorrichtungen können ähnlich zu der einzelnen Sensorvorrichtung vorzugsweise weitere Temperatursensoren angeordnet sein, um die Messpunktanzahl am Finger zu vergrößern und somit mit einer vergrößerten Datenbasis genauere Diagnosen bzw. datenbasierte Handlungsempfehlungen ableiten zu können.

Bei erfindungsgemäßen gekoppelten Sensorvorrichtungen können die mindestens zwei Sensorvorrichtungen so ausgelegt sein, dass eine Sensorvorrichtung die Hauptvorrichtung ist, an die die zweite Sensorvorrichtung die Messdaten zur Speicherung und Übermittlung an eine Auswertungseinheit übermittelt. Auf diese Weise ist eine koordiniertere Übermittlung der Messdaten an die Auswertungseinheit möglich.

Darüber hinaus ist auch die Kopplung der Sensorvorrichtung mit einem Erweiterungsmodul (Handrückenmodul) zur Messung der Temperatur an verschiedenen Punkten auf dem Handrücken in einer Weiterbildung der erfindungsgemäßen Sensorvorrichtung erfindungsgemäß. Die Sensorvorrichtung kann dabei über den am Fingergrundgelenk angeordneten Sensorarm an das Handrückenmodul gekoppelt sein. Das Handrückenmodul kann dabei mit einer Fläche mit verteilten Temperatursensoren auf dem Handrücken zur Anlage kommen, um eine Messpunktanzahl am Handrücken bereitzustellen und somit mit einer vergrößerten Datenbasis genauere Diagnosen bzw. datenbasierte Handlungsempfehlungen erlauben zu können.

Neben der oben genannten Kopplung von zwei oder mehr erfindungsgemäßer Sensorvorrichtungen, ist auch eine Kombination eines oder mehrere erfindungsgemäßer Sensorvorrichtungen mit einer Vorrichtung zur digitalen Messung der Handkraft möglich, welche mit einer der Sensorvorrichtungen mit einem Kabel oder einer kabellosen Datenübertragung verbunden sein kann. Diese Vorrichtung kann in mehreren Ausführungsformen wie folgt ausgebildet sein:
- In Form von zwei gespreizten Kraftaufnehmern, welche jeweils an ihren Enden an einem gemeinsamen Drehpunkt drehbar gelagert. Ein Kraftaufnehmer ist mit seiner Außenfläche anliegend auf der Handinnenfläche, während der andere Kraftaufnehmer mit seiner Außenfläche anliegend an den Fingerinnenflächen angeordnet ist. An dem o.g. Drehpunkt wirkt eine Rückstellmoment entgegen der Handkraft, somit der Kraft, welche mit den Fingern in Richtung der Handinnenfläche aufgebracht wird. Das Rückstellmoment drückt die Kraftaufnehmer auseinander, während die Handkraft die Kraftaufnehmer zusammenbewegt. Durch diese Vorrichtung kann die Kraft in den Händen - genauer die kumulierte Beugekraft der Finger in Richtung der Handinnenfläche - gemessen werden;
- In Form von einem hohlen Volumenkörper, welcher mit einem Innendruck versehen wird. Ein Drucksensor misst in definierten Zeitabständen den Innendruck des Volumenkörpers. Ein Volumenstrom-Durchflussmessgerät misst in definierten Zeitabständen den Volumenstrom an einer direkt am Volumenkörper befestigten Düse. Der Patient wird nach Befüllung des Volumenkörpers durch eine Handpumpe aufgefordert, das Gerät zur Messung der Handkraft so schnell und so fest wie möglich zusammenzudrücken. Durch diese Vorrichtung kann die Kraft in den Händen - genauer die kumulierte Beugekraft der Finger in Richtung der Handinnenfläche - gemessen werden.
- In Form eines schlauchförmigen Körpers mit Dehnungsmessstreifen, welche an einem Finger oder Zeh angebracht werden. In regelmäßigen Abständen in Längsrichtung sind vorzugsweise auf der Außenseite des schlauchförmigen Körpers Dehnungsmessstreifen angebracht, welche eine Dehnung des Schlauches in axialer Richtung messen können und auf die Normalspannungen im Material des schlauchförmigen Körpers Aufschluss geben. Die Dehnung der Dehnungsmessstreifen kommt durch ein Beugen des Messfingers insofern zustande, als dass sich durch die Winkelvergrößerung im Streckvorgang bei gleichem Radius ergebende größere Bogenlänge der Fläche ober dem Fingergelenk eine Dehnung ergibt. Durch diese Vorrichtung kann die Kraft und oder der Bewegungsumfang des Fingers oder Zehs gemessen werden, über den der schlauchförmige Körper zuvor übergestülpt wurde. Dabei kann der schlauchförmige Körper sowohl Luft- und Wasser durchlässig als auch Luft- und Wasser undurchlässig sein

Alle drei Ausführungsformen dieser Vorrichtung können in einer Kombinationsauswertung des Handkraftmessgerätes oder Beweglichkeitsmessgerätes zur Feststellung einer sogenannten Morgensteifigkeit der Finger bzw. einer krankheitsbedingten Kraftlosigkeit verwendet werden. Die Parameter des Handkraftmessgerätes könnten als weitere Datenquelle zu den Temperatur- und Schwellwerten der Finger(gelenke) aus der erfindungsgemäßen Vorrichtung die Therapieempfehlungen, Diagnosen und Prognosen der erfindungsgemäßen Vorrichtung unterstützen.

Neben der oben genannten Kopplung von zwei oder mehr erfindungsgemäßer Sensorvorrichtungen ist auch eine Kombination einer oder mehrerer erfindungsgemäßer Sensorvorrichtungen mit einer Vorrichtung zur allgemeinen Messung der Körpertemperatur des Patienten / Trägers ähnliche einem Fiebermessgerät oder mit einem Handflächentemperaturmessgerät möglich. Dieses Messgerät kann mit einer der Sensorvorrichtungen über ein Kabel oder einer kabellosen Datenübertragung verbunden und kann die Messwerte auswerten. In einer gemeinsamen Auswertung können die Messwerte von Körpertemperaturmessgeräten zur Bestimmung des Allgemeinzustandes des Immunsystems dienen. Dies ist insofern wichtig, da eine Störung des Immunsystems als Ursache für eine rheumatische Arthritis zu einem Überschießen der Immunreaktionen führen kann, welche sich in der Regel in Fieber und Schwitzen äußern kann. Die Messwerte des Körpertemperaturmessgerätes können somit als weitere Datenquellen zusätzliche zu den Temperatur- und Schwellwerten der Finger(-gelenke) der erfindungsgemäßen Vorrichtung die Therapieempfehlungen, Diagnosen und Prognosen der erfindungsgemäßen Vorrichtung unterstützen.

Ferner betrifft die Erfindung auch ein Kit zur Bestimmung und Auswertung von entzündungsrelevanten Vitalparametern, das zumindest eine Sensorvorrichtung und eine Einheit, die zur Auswertung und Speicherung der von der Sensorvorrichtung gewonnenen und gespeicherten Daten und vorzugsweise zur Erstellung einer Therapieempfehlung ausgelegt ist, umfasst, wobei die mindestens eine Sensoreinheit und die Auswertungseinheit miteinander in einer Kommunikationsverbindung gekoppelt sind. Das erfindungsgemäße Kit ermöglicht dem Therapeuten somit die Einleitung von Maßnahmen gegen den Entzündungszustand und somit eine frühzeitige Behandlung der Rheumaschübe und der Diagnose einer rheumatischen Arthritis oder Gicht, etc.

Nachstehend ist ein beispielhaftes Ausführungsbeispiel der erfindungsgemäßen Vorrichtung anhand der schematischen Zeichnungen im Einzelnen erläutert. Dabei zeigt:
Figur 1 ein erstes Ausführungsbeispiel der erfindungsgemäßen Vorrichtung in der Seitenansicht;
Figur 2A eine Querschnittsansicht entlang der Ebene A - A durch die in Figur 1 gezeigte Vorrichtung;
Figur 2C eine Detailansicht der in Figur 1 umkreisten Detailansicht C;

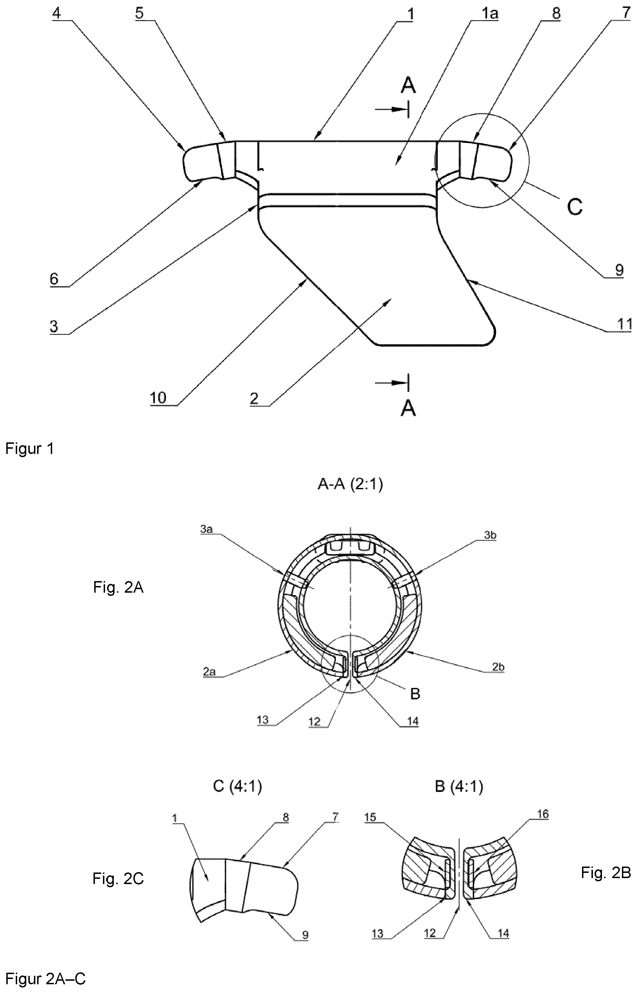

Figur 2B eine in Figur 2A umkreiste Detailansicht B;

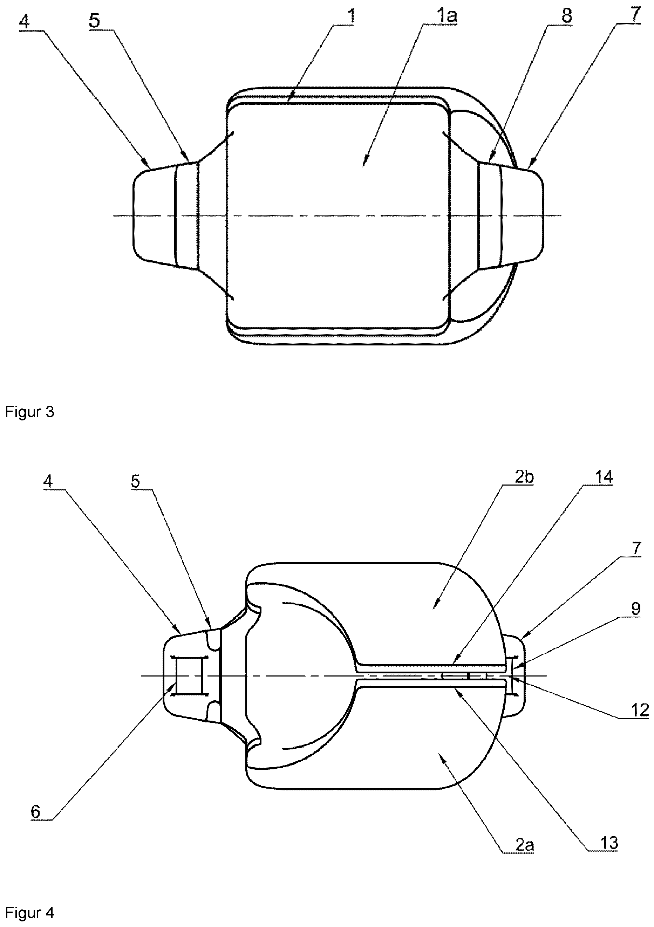

Figur 3 eine Draufsicht auf die erfindungsgemäße Vorrichtung gemäß Figur 1 von oben;
Figur 4 eine Ansicht der erfindungsgemäßen Vorrichtung gemäß Figur 1 von unten;

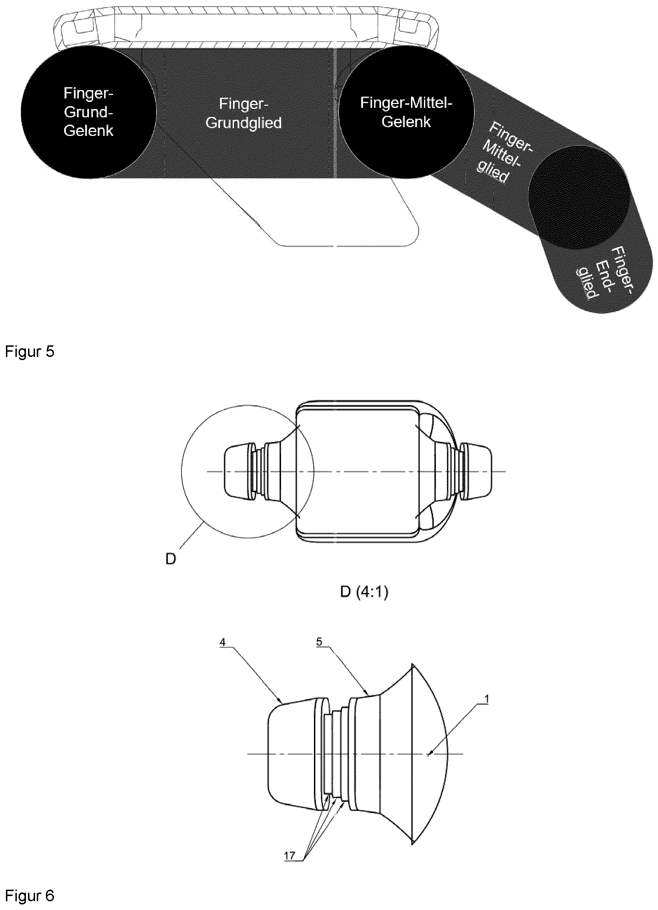

Figur 5 eine schematische Darstellung der auf einen Finger aufgesteckten erfindungsgemäßen Vorrichtung gemäß Figur 1;
Figur 6 eine weitere Ausführungsform der erfindungsgemäßen Vorrichtung mit teleskopartigen Zwischenelementen, optional als Federelemente ausgeführt;

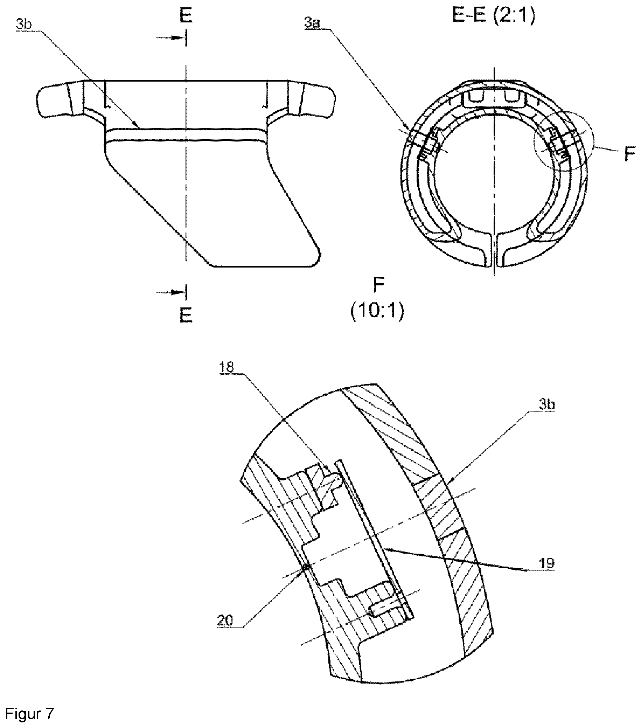

Figur 7 eine weitere Ausführungsform der erfindungsgemäßen Vorrichtung zur Messung der Änderung der Fingerdicke;

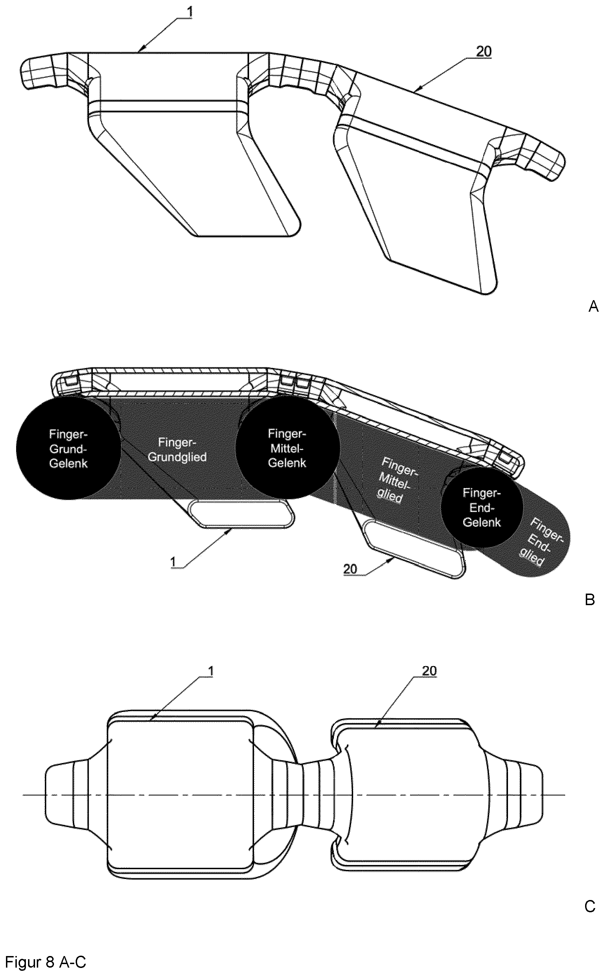

Figur 8 A-C eine erfindungsgemäße Vorrichtung mit zwei miteinander gekoppelten Sensorvorrichtungen in einer Seitenansicht, in einer schematischen Ansicht der Anordnung am Finger sowie in einer Draufsicht von oben;

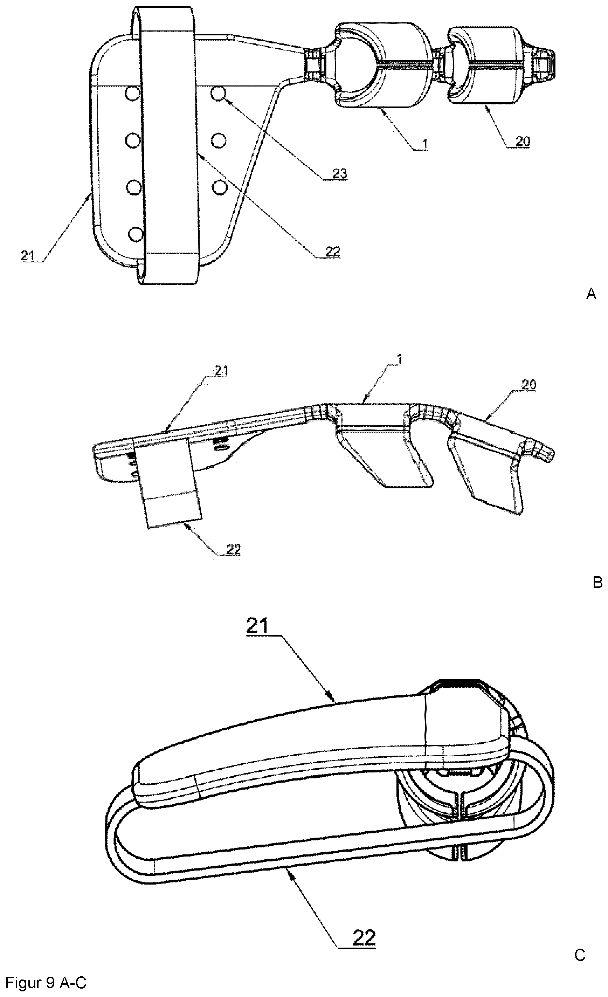

Figur 9 A-C ein erfindungsgemäßes Sensorvorrichtungssystem mit zwei miteinander gekoppelten Sensorvorrichtungen und einem daran gekoppelten Handrückenmodul in einer Draufsicht von unten, in einer Seitenansicht sowie in einer perspektivischen Draufsicht von oben; und

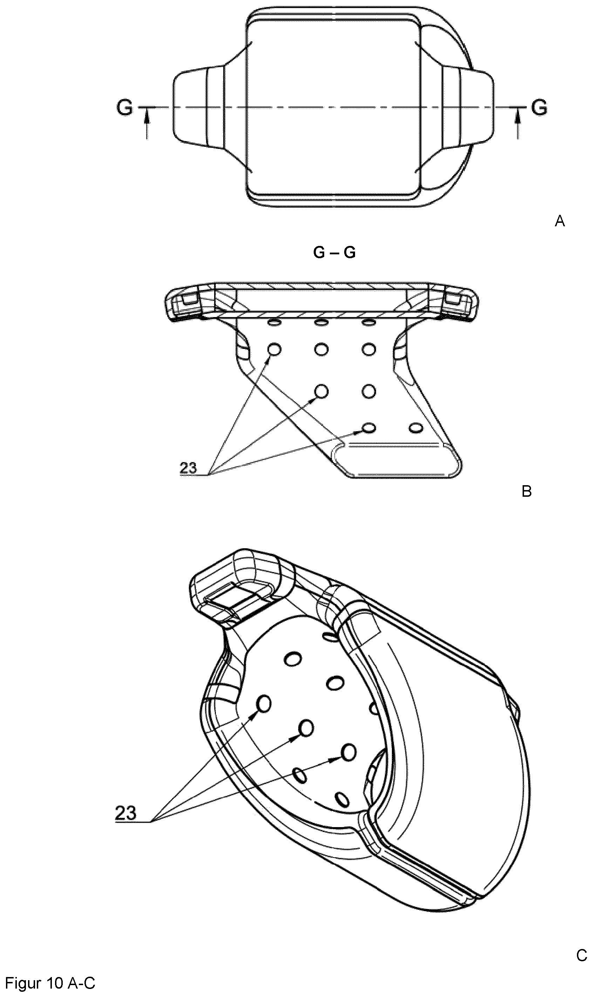

Figur 10 A-C eine erfindungsgemäße Vorrichtung mit in den Ringsegmenten angeordneten Temperatursensoren in einer Draufsicht von oben, in einer Querschnittsansicht entlang der in der Figur 10 A angegebenen Linie G_G, in einer perspektivischen Draufsicht von unten.

Wie in Figur 1 gezeigt, umfasst die erfindungsgemäße Sensorvorrichtung 1 in der gezeigten Ausführungsform einen Grundkörper 1A mit daran beidseits angesetzten Ringsegmenten/Halbringen 2A, 2B, von denen zumindest einer über ein flexibles, Zwischenelement 3 als Federelement mit dem Grundkörper 1A verbunden ist.

In einer Ausführungsform der erfindungsgemäßen Sensorvorrichtung 1 sind beidseitig des Grundkörpers 1A Halbringe (2A, 2B) über die Zwischenelemente als Federelemente 3 (3A, 3B) angesetzt. Die Federelemente 3 sind so ausgelegt, dass die erfindungsgemäße Vorrichtung 1 im auf den Finger aufgesteckten Zustand mit den Halbringen 2A, 2B am Finger beidseits anliegt und den Spalt 12 ausbilden. Die Halbringe 2A, 2B sind im Radius der Flächen des hohlen Halbzylinders so bemessen oder können so ausgestaltet sein, dass eine an den jeweiligen. Fingerdurchmesser des Trägers angepasste Ausführung der erfindungsgemäßen Sensorvorrichtung möglich ist. Die Halbringe 2A, 2B sind an der, dem Fingergrundgelenk in der Gebrauchsposition zugewandten Vorderkante 10 in Richtung zu der dem Grundkörper 1A gegenüberliegenden Seite zu den Halbringenden 13 und 14 jeweils so angeschrägt, dass bei Aufstecken der erfindungsgemäßen Sensorvorrichtung 1 auf den Finger in der Gebrauchsposition der Temperatursensor 6, der am Sensorarm 4 über das Zwischenelement 5 am Grundkörper 1 festgelegt auf dem Fingergrundgelenk zur Anlage kommt. Dabei ist der Sensorarm 4 und/oder das Zwischenelement 5 bevorzugt so ausgestaltet, dass es der Länge des Fingergliedes angepasst ist oder der Fingergliedlänge des Patienten Rechnung tragend in Fingergliedrichtung auf das Grundgelenk in Gebrauchsposition hin in der Länge nach Art eines Teleskopes längenverstellbar ist. Neben der Ausgestaltung des Zwischenelementes 5 derart, dass die Temperaturmessfläche des Temperatursensors 6 auf dem Fingergrundgelenk anliegt, können zusätzlich an dem Sensorarm 4 zusätzlich in der Figur nicht dargestellte Bauelemente vorhanden sein, die den Finger zumindest teilweise umgreifen und einen sicheren Anpressdruck für die Temperaturmessfläche des Temperatursensors 6 auf dem Fingergrundgelenk gewährleisten oder diese unterstützen. Auf der, dem Fingergrundgelenk in der Gebrauchsposition abgewandten Seite jedes Halbringes 2A, 2B kann jeder Halbring 2A, 2B eine abgeschrägte Hinterkante 11 aufweisen, die in Richtung des Fingermittelgelenkes in Gebrauchsposition angeschrägt ist. Durch die so vergrößerte axiale Länge jedes Halbringes ist in der Gebrauchsposition ein zuverlässiger und kippneigungsfreier Sitz der Sensorvorrichtung 1 auf dem Finger gewährleistet. An der, dem Fingergrundgelenk in Gebrauchsposition abgewandten Seite des Grundkörpers 1A der Sensorvorrichtung 1 kann ähnlich wie auf der in der Gebrauchsposition dem Fingergrundgelenk zugewandten Seite ein Sensorarm 7 vorgesehen sein, der über ein Zwischenelement 8 mit dem Grundkörper verbunden ist und an seiner dem Fingermittelgelenk zugewandten Seite zumindest einen Temperatursensor 9 aufweist, das in Gebrauchsposition auf dem Fingermittelgelenk zur Anlage kommt. Der Sensorarm 7 und/oder das Zwischenelement 8 sind in der axialen Länge so bemessen oder können so teleskopartig ausgestaltet sein, dass eine sichere Anlage des Temperatursensors 9 auf dem Fingermittelgelenk ermöglicht wird.

Im Grundkörper 1A sind Mittel zur Speicherung und Übertragung der von dem/den Temperatursensor(en) gemessenen Daten auf eine in den Figuren nicht gezeigte Datenauswerteeinheit vorgesehen, wobei die Temperatursensoren über eine oder mehrere Verbindungen, beispielsweise Kabelverbindungen, mit den Mitteln zur Speicherung in kommunikativer Verbindung stehen. Die Datenübertragung auf die Auswertungseinheit kann über eine in der Zeichnung nicht dargestellte Kabelverbindung oder drahtlos erfolgen. Eine drahtlose Übertragung bietet sich vorteilhaft über eine NFC-Verbindung oder eine Bluetooth-Verbindung an. Zur Stromversorgung der Elemente für die Temperaturmessung, Datenspeicherung und Datenübertragung kann in den Grundkörper 1 eine Batterie wie z.B. eine an der Halbzylinderform angepasste Lithium-Ionen-Batterie integriert sein. Alle Schnittstellen für einen möglichen o.g. Kabelanschluss sind mit einem schnittstellenkontur angepassten Blindstopfen versehen, welche einer Schmutz- und Wassereindringung in den Grundkörper entgegenwirken soll.

Wie in den Figuren 2A-2C gezeigt, sind die Halbringe 2A und 2B an den dem Grundkörper gegenüberliegenden Halbringunterkanten 13 und 14 über einen Spalt 12 beabstandet. Wie in Figur 2B gezeigt, kann in die Halbringunterkanten 13 und 14 jeweils eine durch den Spalt 12 getrennte Fläche 15, 16 aus einem leitfähigem Metall integriert sein, an das Spannung angelegt wird und eine Art Kondensator ausbildet. Bei Vergrößerung des Spaltes 12 wird eine Potentialänderung an den Metallflächen 15 und 16 nach Art eines Kondensators erzeugt, die die Vergrößerung des Spaltes und somit die Zunahme der Fingerdicke berechnen lässt.

Wie in Figur 2C in vergrößerter Ansicht gezeigt, kann der Grundkörper ein Zwischenelement 8 sowie den Sensorarm 7 und das dem Fingermittelgelenk zugewandte Sensorelement 9 aufweisen, das in Gebrauchsposition auf dem Fingermittelgelenk zur Anlage kommt.

In Figur 3 ist die erfindungsgemäße Sensorvorrichtung 1 in der Draufsicht von oben gezeigt. Der Sensorarm 4 ist dabei über das Zwischenelement 5 mit dem Grundkörper 1A verbunden. Auf der dem Fingergrundgelenk in Gebrauchsposition gegenüberliegenden Seite des Grundkörpers, die in der Gebrauchsposition dem Fingermittelgelenk zugewandt ist, ist der Sensorarm 7 über das Armefederelement 8 mit dem Grundkörper 1A verbunden.

In Figur 4 ist die erfindungsgemäße Vorrichtung in der Draufsicht von unten gezeigt. Die Halbringe 2A und 2B sind an ihren dem Grundkörper gegenüberliegende Unterkanten 13 und 14 über den Spalt 12 voneinander beabstandet. Der Sensorarm 4, der über das Zwischenelement 5 am Grundkörper angeordnet ist, weist ein dem Finger im Gebrauchszustand zugewandtes Temperatursensor 6 auf. In entsprechender Weise weist der über das in der Zeichnung nicht dargestellte Zwischenelement am Grundkörper festgelegte Sensorarm 7 die Temperatursensor 9 auf, die in der Gebrauchsposition auf dem Fingermittelgelenk aufliegt.

Erfindungsgemäß ist bei Ausführung des Zwischenelementes als ein Federelement 3, 5, 8 im Allgemeinen aus einem flexiblen Kunststoff, Metall oder Nichtmetall gefertigt, bei dem gegen eine Auslenkung aus der Normalposition, auch unter einer Grundvorspannung, eine Rückstellkraft so wirkt, die Normalposition wieder einzunehmen. Auf diese Weise wird erreicht, dass der Sensorarm und der daran angeordnete Temperatursensor, insbesondere auch bei Bewegung des Fingers, in der Gebrauchsposition auf dem Gelenk aufliegt und je nach Grundvorspannung an das Gelenk gepresst wird. In ähnlicher Weise liegen die Halbringe an dem Fingerglied unter Einwirkung des zumindest einen Zwischenelementes in Form eines Armfederelementes an dem Fingerglied so an, dass ein zuverlässiger Sitz der erfindungsgemäßen Vorrichtung auf dem Fingerglied gewährleistet ist und gleichzeitig weitere physiologische Parameter bestimmt werden können. Dazu können in den Halbringen weitere Sensoren angeordnet sein, die beispielsweise zur Bestimmung der Leitfähigkeit der Haut, der Sauerstoffsättigung des Blutes und weiterer Parameter wie Puls und Herzrhythmus, der Konzentration der Erythrozyten, der Konzentration der Leukozyten, der Konzentration der Lymphozyten und der Konzentration von Hämoglobin (Hb) dienen können.

In Figur 5 ist die Anordnung der erfindungsgemäßen Vorrichtung auf einem Finger schematisch dargestellt. Eine auf den Finger aufgesteckte erfindungsgemäße Vorrichtung ist am Fingergrundglied so angeordnet, dass die Temperatursensoren auf dem Fingergrundgelenk, bzw. auf dem Fingermittelgelenk aufliegen. Auf diese Weise kann eine sichere und optionale differenzielle Temperaturmessung an beiden Gelenken durchgeführt werden und die Messdaten zur Auswertung an eine Auswertungseinheit übertragen werden. Gleichzeitig kann im Falle eines Rheumaschubs oder zur Prognose eines Rheuma-Schubes die Zunahme der Fingerdicke über die Halbringe 13 und 14 und deren Spaltabstand bestimmt werden.

In Figur 6 ist eine Ausführungsform der erfindungsgemäßen Vorrichtung dargestellt, bei der die Sensorarme 4 und 7 über je ein Zwischenelement 5 und 8 mit Teleskopgliedern 17 am Grundkörper lösbar festgelegt sind, die eine Anpassung der Sensorarmlänge an die physiognomischen Verhältnisse beim Patienten erlauben.

Figur 7 zeigt eine weitere Ausführungsform zur Messung der Auslenkung des/der Federelemente/s mit einer Seitenansicht auf die erfindungsgemäße Sensorvorrichtung, einer Schnittansicht entlang der Ebene E-E sowie einer Detailansicht F. In dieser Ausführungsform misst ein Kraftaufnehmer/Kraftsensor 18 eine auf ihn durch einen Biegestab / Biegeblech 19 aufgebrachte Kraft. Diese Anordnung entspricht der eines einseitig eingespannten Trägers. Die Querauslenkung des Trägers verhält sich proportional zur Kraft, welche an der Anlagefläche am Kraftaufnehmer (18) gemessen wird. Die Querauslenkung ist auch proportional zur Auslenkung des Federelements (3A/3B). Im Kraftaufnehmer 18 wandeln Piezoelemente die Kraft in eine Spannungsänderung um, welche über einen Analog-Digital-Konverter verarbeitet wird. Aus der Spannungsänderung wird die Kraft bestimmt und daraus die Änderung der Fingerdicke.

Figuren 8 A-C zeigen eine erfindungsgemäße Vorrichtung mit zwei miteinander gekoppelten Sensorvorrichtungen (1;20) in einer perspektivischen Draufsicht, in einer schematischen Ansicht der Anordnung am Finger sowie in einer Draufsicht von oben. Bei den erfindungsgemäßen Vorrichtungen, die mindestens eine Sensoreinheit wie die Sensorvorrichtungen (1;20) am Finger und/oder ein Handrückenmodul betreffen, können die Übergänge zwischen den Bauteilen ähnlich den Zwischenelementen (5) teleskopartig ausgebildet sein, so dass eine Längenanpassung der Übergangsbereiche der erfindungsgemäßen Sensorvorrichtung an unterschiedliche Fingerlängen und/oder Handgrößen ermöglicht wird.

Figuren 9 A-C zeigen ein erfindungsgemäßes Sensorvorrichtungssystem mit zwei miteinander gekoppelten Sensorvorrichtungen (1;20) und einem daran gekoppelten Handrückenmodul (21) mit einem Fixierband (22) und Sensoren (23), beispielsweise Temperatursensoren oder Sensoren für unterschiedliche Parameter, in einer Draufsicht von unten, in einer Seitenansicht sowie in einer perspektivischen Draufsicht von oben, jeweils in einer Ausführung für den linken Zeigefinger. Bei Ausführungen für andere Finger kann das Handrückenmodul (21) in seiner Form entsprechend angepasst werden.

Figuren 10 A-C zeigen eine erfindungsgemäße Sensorvorrichtung (1) mit in den Ringsegmenten (2A;2B) angeordneten Sensoren (23), beispielsweise Temperatursensoren, in einer Draufsicht von oben, in einer Querschnittsansicht entlang der in der Figur 10 A angegebenen Linie G_G, und in einer perspektivischen Draufsicht von unten.

### Bezugszeichenliste

- 1: Sensorvorrichtung
- 1A: Grundkörper
- 2: Ringförmiger Halter (2) mit Ringsegment (2A, 2B)
- 3: Federelement (3A, 3B)
- 4: Sensorarm
- 5: Zwischenelement
- 6: Temperatursensor
- 7: Sensorarm
- 8: Zwischenelement
- 9: Temperatursensor
- 10: Vorderkante
- 11: Hinterkante
- 12: Spalt
- 13: Ringsegmentunterkante
- 14: Ringsegmentunterkante
- 15: Sensorfläche
- 16: Sensorfläche
- 17: Teleskopglieder
- 18: Kraftsensor
- 19: Biegestab
- 20: Sensorvorrichtung Mittelglied (analog zu Pos 1)
- 21: Handrückenmodul
- 22: Fixierband
- 23: Sensor

## Patentansprüche

1. Sensorvorrichtung (1) zur Bestimmung von entzündungsrelevanten Vitalparametern, mit einem Grundkörper (1a) mit einer im Wesentlichen rechteckigen Grundfläche, einem am Grundkörper (1a) festgelegten ringförmigen Halter (2) zum Aufstecken auf einen Finger eines Patienten und mindestens einem am Grundkörper (1a) lösbar festgelegten Sensorarm (4), wobei eine einem Temperatursensor (6) funktionell zugeordnete Sensorfläche am Sensorarm (4) endständig so angeordnet ist und der Sensorarm (4) eine solche Länge aufweist, dass die Sensorfläche in der Gebrauchsposition auf dem Fingergrundgelenk des Patienten zur Anlage kommt, wobei im Grundkörper Mittel zur Speicherung und zur optionalen Übertragung der von dem Temperatursensor gemessenen Daten auf eine Datenauswerteeinheit vorgesehen sind, und wobei der mindestens eine am Grundkörper (1a) festgelegte Sensorarm (4) über ein vorzugsweise flexibles Zwischenelement (5) am Grundkörper lösbar festgelegt ist.

2. Sensorvorrichtung (1) zur Bestimmung von entzündungsrelevanten Vitalparametern nach Anspruch 1, wobei am Grundkörper (1a) ein weiterer Sensorarm (7) lösbar optional über ein Zwischenelement (8) so festgelegt ist, dass eine einem Temperatursensor (9) funktionell zugeordnete Sensorfläche am Sensorarm (7) endständig so angeordnet ist und der Sensorarm (7) eine solche Länge aufweist, dass die Sensorfläche (9) in der Gebrauchsposition auf dem Fingermittelgelenk des Patienten zur Anlage kommt.

3. Sensorvorrichtung (1) zur Bestimmung von entzündungsrelevanten Vitalparametern nach Anspruch 1 oder 2, wobei der Sensorarm (4) und/oder der Sensorarm (7) zu der durch die Grundfläche des Grundkörpers (1a) aufgespannten Ebene einen Winkel von 0° bis 45°, insbesondere 20° bis 30° in Richtung des ringförmigen Halters 2 einschließt.

4. Sensorvorrichtung (1) zur Bestimmung von entzündungsrelevanten Vitalparametern nach einem der Ansprüche 1 bis 3, wobei der ringförmige Halter 2 zwei Ringsegmente (2A, 2B) umfasst, von denen zumindest eines über ein Federelement (3) am Grundkörper (1A) festgelegt ist.

5. Sensorvorrichtung (1) zur Bestimmung von entzündungsrelevanten Vitalparametern nach Anspruch 4, wobei der ringförmige Halter (2) zwei Ringsegmente (2A, 2B) umfasst, die jeweils über ein Federelement (3A,3B) am Grundkörper (1A) festgelegt sind und an dem, dem Grundkörper (1A) gegenüberliegenden Ende einen Spalt (12) ausbilden, wobei die Ringsegmente (2A, 2B) jeweils an ihrem dem Spalt (12) zugewandten Ende (13, 14) mindestens einen Sensor zur Bestimmung der Spaltbreite aufweisen.

6. Sensorvorrichtung (1) zur Bestimmung von entzündungsrelevanten Vitalparametern nach einem der vorhergehenden Ansprüche, wobei in dem ringförmigen Halter (2) oder dem Grundkörper (1A) zumindest ein weiterer Sensor (23) zur Bestimmung eines physiologischen Parameters, ausgewählt aus der Leitfähigkeit der Haut, der Sauerstoffsättigung des Blutes, dem Puls, dem Herzrhythmus, der Konzentration von Blutzellen oder der Konzentration von Hämoglobin (Hb), angeordnet ist.

7. System zur Bestimmung von entzündungsrelevanten Vitalparametern, umfassend zumindest eine oder bevorzugt mindestens zwei Sensorvorrichtungen (1) nach einem der Ansprüche 1 bis 6 und optional ein Handrückenmodul (21) mit Sensoren (23), die miteinander zur Übertragung der von der jeweiligen Sensorvorrichtung gewonnenen und gespeicherten Daten in einer Kommunikationsverbindung gekoppelt sind.

8. Kit zur Bestimmung und Auswertung von entzündungsrelevanten Vitalparametern, umfassend zumindest eine Sensorvorrichtung nach einem der Ansprüche 1 bis 6 und eine Einheit, die zur Auswertung und Speicherung der von der Sensorvorrichtung gewonnenen und gespeicherten Daten und vorzugsweise zur Erstellung einer Therapieempfehlung oder einer Verhaltensempfehlung für den Patienten ausgelegt ist, wobei die mindestens eine Sensoreinheit und die Auswertungseinheit miteinander in einer Kommunikationsverbindung gekoppelt sind.

## Claims

1. Sensor device (1) for determining vital parameters relevant to inflammation, having a base body (1a) with a substantially rectangular base surface, an ring-shaped holder (2), which is fixed to the base body (1a), for placing on a finger of a patient and at least one sensor arm (4), which is detachably fixed to the base body (1a), wherein a sensor surface, which is functionally associated with a temperature sensor (6), is arranged at the end on the sensor arm (4) in such a way and the sensor arm (4) has such a length that the sensor surface in the position of use comes to rest on the base joint of the patient's finger, wherein means for storing and optionally transmitting the data measured by the temperature sensor to a data evaluation unit are provided in the base body, and wherein the at least one sensor arm (4) fixed to the base body (1a) is detachably fixed to the base body via a preferably flexible intermediate element (5).

2. Sensor device (1) for determining vital parameters relevant to inflammation according to claim 1 , wherein a further sensor arm (7) is detachably fixed optionally via an intermediate element (8) to the base body (1a) in such a way that a sensor surface functionally associated with a temperature sensor (9) is arranged at the end of the sensor arm (7) in such a way and the sensor arm (7) has such a length that the sensor surface (9) comes to rest on the patient's finger middle joint in the position of use.

3. Sensor device (1) for determining vital parameters relevant to inflammation according to claim 1 or 2, wherein the sensor arm (4) and/or the sensor arm (7) encloses an angle of 0° to 45°, in particular 20° to 30°, in the direction of the ring-shaped holder 2 with respect to the plane spanned by the base surface of the base body (1a).

4. Sensor device (1) for determining vital parameters relevant to inflammation according to one of the claims 1 to 3 , wherein the ring-shaped holder 2 comprises two ring segments (2A, 2B), at least one of which is fixed to the base body (1A) via a spring element (3).

5. Sensor device (1) for determining vital parameters relevant to inflammation according to claim 4, wherein the ring-shaped holder (2) comprises two ring segments (2A, 2B) which are each fixed to the base body (1A) via a spring element (3A, 3B) and form a gap (12) at the end opposite the base body (1A), (1A) and form a gap (12) at the end opposite the base body (1A), the ring segments (2A, 2B) each having at least one sensor for determining the gap width at their end (13, 14) facing the gap (12).

6. Sensor device (1) for determining vital parameters relevant to inflammation according to one of the preceding claims, wherein at least one further sensor (23) for determining a physiological parameter selected from the conductivity of the skin, the oxygen saturation of the blood, the pulse, the heart rhythm, the concentration of blood cells or the concentration of haemoglobin (Hb) is arranged in the ring-shaped holder (2) or the basic body (1A).

7. System for determining vital parameters relevant to inflammation, comprising at least one or preferably at least two sensor devices (1) according to any one of claims 1 to 6 and optionally a back-of-hand module (21) with sensors (23) which are coupled to one another for transmitting the data obtained and stored by the respective sensor device in a communication link.

8. Kit for determining and evaluating vital parameters relevant to inflammation, comprising at least one sensor device according to one of claims 1 to 6 and a unit which is designed for evaluating and storing the data obtained and stored by the sensor device and preferably for preparing a therapy recommendation or a behaviour recommendation for the patient, wherein the at least one sensor unit and the evaluation unit are coupled to one another in a communication link.

## Revendications

1. Ensemble capteur (1) destiné à déterminer des paramètres vitaux relatifs à des inflammations, comportant un corps de base (1a) qui présente une surface de base sensiblement rectangulaire, une attache (2) annulaire rapportée au corps de base pour être emboîtée sur le doigt d'un patient et au moins un bras de capteur (4) susceptible d'être rapporté au corps de base (1a) de manière amovible, une surface de capteur associée fonctionnellement à un capteur de température (6) étant disposée à l'extrémité du bras de capteur (4) et le bras de capteur (4) présentant une longueur telle qu'elle permet l'appui de la surface de capteur sur l'articulation métacarpo-phalangienne du patient en position d'utilisation, le corps de base présentant des moyens de stockage et de transmission facultative des données mesurées par le capteur de température à une unité de dépouillement des données, et l'au moins un bras de capteur (4) rapporté au corps de base (1a) étant fixé de manière amovible au corps de base (5) via un élément intermédiaire (5) de préférence flexible.

2. Ensemble capteur (1) destiné à déterminer des paramètres vitaux relatifs à des inflammations selon la revendication 1, dans lequel, en option, un bras de capteur (7) supplémentaire est rapporté de manière amovible au corps de base (1a) via un élément intermédiaire (8) de sorte qu'une surface de capteur associée fonctionnellement à un capteur de température (9) est disposée à l'extrémité du bras de capteur (7) et le bras de capteur (7) présentant une longueur telle qu'elle permet l'appui de la surface de capteur (9) sur l'articulation intermédiaire du doigt du patient en position d'utilisation.

3. Ensemble capteur (1) destiné à déterminer des paramètres vitaux relatifs à des inflammations selon la revendication 1 ou 2, dans lequel le bras de capteur (4) et / ou le bras de capteur (7) renferme/nt un angle de 0 à 45°, notamment de 20° à 30° en direction de l'attache annulaire (2) par rapport au plan défini par la surface de base du corps de base (1a).

4. Ensemble capteur (1) destiné à déterminer des paramètres vitaux relatifs à des inflammations selon l'une des revendications 1 à 3, dans lequel l'attache annulaire (2) présente deux segments annulaires (2a, 2b) dont l'un au moins est rapporté au corps de base (1a) via un élément à ressort (3).

5. Ensemble capteur (1) destiné à déterminer des paramètres vitaux relatifs à des inflammations selon la revendication 4, dans lequel l'attache annulaire (2) comporte deux segments annulaires (2a, 2b) rapportés chacun au corps de base (1a) via un élément à ressort (3a, 3b) et formant, à l'extrémité opposée au corps de base (1a) une fente (12), les éléments annulaires (2a, 2b) présentant chacun à leur extrémité (13, 14) opposée à la fente (12) au moins un capteur de détermination de la largeur de la fente.

6. Ensemble capteur (1) destiné à déterminer des paramètres vitaux indicatifs d'inflammations selon l'une des revendications précédentes, dans lequel, dans l'attache annulaire (2) ou dans le corps de base (1a), au moins un capteur (23) supplémentaire est prévu pour déterminer un paramètre physiologique, choisi parmi les paramètres suivants: conductivité de la peau, oxygénation du sang, pouls, rythme cardiaque, concentration ce cellules sanguines ou concentration d'hémoglobine (Hb).

7. Système de détermination de paramètres vitaux relatifs à des inflammations, comportant au moins un, de préférence deux ensembles capteurs (1) selon l'une des revendications 1 à 6 et, en option, un module de dos de main (21) comportant des capteurs (23) reliés par une connexion de transmission pour transmettre les données récupérées de l'ensemble capteur correspondant et stockées dans celui-ci.

8. Kit de détermination et de dépouillement de paramètres vitaux relatifs à des inflammations comportant au moins un ensemble capteur selon l'une des revendications 1 à 6, ainsi qu'une unité conçue pour dépouiller et stocker les données récupérées de l'ensemble capteur et stockées dans celui-ci et, de préférence, pour préparer une recommandation thérapeutique ou une recommandation de comportement pour le patient, l'au moins un ensemble capteur et l'unité de dépouillement étant reliés par une connexion de transmission.
